# FASCICULE DE BREVET EUROPEEN

(11) **EP 0 709 084 B1**
(45) Date de publication et mention de la délivrance du brevet: **02.12.1998**
(21) Numéro de dépôt: 95401968.3
(22) Date de dépôt: 28.08.1995
(51) Int. Cl.: A61K 7/48, A61K 35/78

(54) **Utilisation d'une huile riche en acide pétrosélinique comme agent hydratant**
Verwendung eines an Petroselinsäurereichen Öls als Feuchthaltemittel
Use of an oil rich in petroselinic acid as hydrating agent

(30) Priorité: 07.10.1994 FR 9412006
(43) Date de publication de la demande: 01.05.1996
(73) Titulaire: L'OREAL, 75008 Paris (FR)
(72) Inventeur: Laugier, Jean-Pierre, F-92160 Antony (FR); Picard, Elisabeth, F-78140 Velizy (FR)
(74) Mandataire: Dodin, Catherine

(56) Documents cités:
- EP-A- 0 556 424
- SEIFEN-ÖLE-FETTE-WACHSE, vol. 113, no. 10, 25 Juin 1987, AUGSBURG, DE, page 342 XP002032285 "Sicherheit und Rohstoffe"
- DATABASE WPI Week 9512 Derwent Publications Ltd., London, GB; AN 95-087989 XP002032287 & RU 2 014 822 A (KOMITEKS FIRM)
- STN, Serveur de Bases de Données, XP002032286

## Description

L'invention se rapporte à une utilisation d'une composition cosmétique ou dermatologique contenant une huile riche en acide pétrosélinique, pour le traitement de la peau, y compris le cuir chevelu, notamment pour l'hydratation de la peau, et en particulier pour le traitement des peaux sèches.

La plupart des compositions cosmétiques et/ou dermatologiques contiennent, à une concentration plus ou moins élevée, une phase grasse généralement constituée d'au moins une huile, une matière grasse et/ou une cire, par exemple sous forme d'émulsions du type eau-dans-huile ou huile-dans-eau, de gels huileux, de solutions huileuses et de produits de maquillage.

Les huiles constituent généralement une proportion importante de cette phase grasse et peuvent être d'origine très variée. Il peut s'agir notamment d'huiles végétales, animales, minérales ou encore d'huiles synthétiques.

Parmi ces huiles, les huiles végétales constituent une classe de choix car la tendance actuelle est plus particulièrement orientée vers l'utilisation de produits d'origine naturelle. Ces huiles végétales sont généralement constituées de triglycérides d'acides gras.

La demanderesse a maintenant trouvé qu'il était possible d'obtenir des compositions cosmétiques ou dermatologiques ayant d'excellentes propriétés, et en particulier des propriétés d'hydratation, contenant une huile végétale riche en acide pétrosélinique extraite de graines d'ombellifères, en particulier de graines de coriandre.

La présente invention a donc pour objet une utilisation d'une huile extraite de la graine de coriandre, comme agent hydratant dans une composition cosmétique et/ou dermatologique.

On entend par huile riche en acide pétrosélinique une huile contenant au moins 40 % d'acide pétrosélinique.

Une telle huile n'a, jusqu'à présent, jamais été préconisée pour constituer un corps gras dans une composition cosmétique ou dermatologique, notamment comme actif pour hydrater la peau.

Certes, il est connu d'utiliser dans le domaine cosmétique des huiles essentielles, y compris des huiles essentielles d'ombellifères (voir Handbook of cosmetic Science and technology, 1st Edition, page 324). Les huiles essentielles sont des produits odoriférants obtenus à partir d'une matière végétale, généralement par entraînement à la vapeur ou par extraction par un solvant. Elles sont donc utilisées dans les produits cosmétiques pour y apporter une odeur. Ainsi, le document GB-A-2262887 divulgue l'utilisation d'huile essentielle de coriandre dans les déodorants et le document EP-A-248701 décrit un produit pour le traitement de la peau, contenant, entre autres substances odoriférantes, de l'huile essentielle de coriandre.

Chimiquement, les huiles essentielles se différencient tout à fait des huiles végétales : elles ne sont pas des corps gras et en particulier ne contiennent pas de triglycérides d'acides gras. Elles sont essentiellement constituées de terpènes aliphatiques et cycliques, de terpénoïdes oxygénés et de composés aromatiques et/ou hétérocycliques. Ainsi, par exemple l'huile essentielle de coriandre est une essence riche en alcools terpéniques, et notamment en linalol avec un peu de géraniol et de bornéol.

Par ailleurs, le document JP-A-60199807 divulgue l'utilisation, comme hydratant, d'un extrait d'ombellifères. Il s'agit d'un extrait hydroalcoolique qui est hydrosoluble, et donc exempt de matières grasses, et qui se différencie nettement d'une huile qui, par définition, est un corps gras lipophile.

Par ailleurs, on sait que l'acide pétrosélinique est un acide gras, l'acide cis-6-octadécènoïque, connu comme inhibiteur de l'absorption lipidique dans le domaine pharmaceutique (voir par exemple le document EP-A-462290). En outre, le document EP-A-116439 divulgue que certains acides gras, dont l'acide pétrosélinique, ont une forte activité d'inhibition de la 5α-réductase et peuvent être utilisés de ce fait dans les produits toniques pour le traitement des cheveux. Ces documents ne décrivent ni ne suggèrent que l'acide pétrosélinique puisse avoir des propriétés particulières, notamment des propriétés hydratantes, intéressantes pour le traitement la peau. L'acide pétrosélinique y est au contraire cité parmi d'autres acides gras ne présentant pas ces propriétés comme le montre le test décrit ci-dessous.

Par ailleurs, aucun de ces documents ne suggère ni ne décrit l'utilisation des triglycérides d'acide pétrosélinique, qui constituent l'huile d'ombellifère selon l'invention.

Les ombellifères sont des plantes dont les fleurs sont disposées en ombelles. On peut citer comme exemples d'ombellifères le coriandre, le cerfeuil, le persil, la carotte, le céleri, le fenouil, le cumin et l'aneth.

L'huile de coriandre utilisée selon l'invention est extraite de la graine de cette ombellifère, par exemple par broyage ou pressage, puis raffinage.

L'huile d'ombellifère a une teneur en acide pétrosélinique qui varie selon la graine d'ombellifère d'où elle est extraite. Pour une même ombellifère, la teneur en acide pétrosélinique varie aussi selon le pays d'origine de l'ombellifère et selon l'extraction qui peut être plus ou moins complète. Ainsi, l'huile de graines de coriandre comporte environ de 75 à 90 % d'acide pétrosélinique, l'huile de graines de cumin en comporte environ de 55 à 65 %, l'huile de graines de carotte environ de 55 à 65 %, l'huile de graines de persil environ de 40 à 50 %, l'huile de graines de fenouil environ de 55 à 65 %, l'huile de graines de cerfeuil environ de 50 à 70 %, l'huile de graines de céleri environ de 45 à 60 %, l'huile de graines d'aneth environ de 75 à 85 %, ces pourcentages étant des pourcentages en poids par rapport au poids total de la composition.

La demanderesse a trouvé de manière inattendue que l'huile de graine de coriandre selon l'invention présentait des propriétés hydratantes.

Aussi, l'invention a également pour objet l'utilisation d'une huile extraite de la graine de coriandre pour préparer une pommade ou un onguent dermatologique destiné au traitement thérapeutique de la peau sèche.

L'invention a encore pour objet un procédé pour hydrater la peau consistant à appliquer sur celle-ci une composition cosmétique contenant une huile extraite de la graine de coriandre.

On sait qu'il est important, notamment pour éviter le vieillissement de la peau, de maintenir une bonne hydratation cutanée en vue de compenser la deshydratation que subit la peau exposée à l'atmosphère. L'huile selon l'invention est donc particulièrement intéressante du fait de ses propriétés hydratantes.

L'huile de coriandre peut par exemple être utilisée dans la composition selon l'invention en une concentration allant de 0,01 % à 50 % en poids par rapport au poids total de la composition, et de préférence de 0,2 % à 20 % en poids par rapport au poids total de la composition.

La composition utilisée selon l'invention peut se présenter sous toutes les formes galéniques normalement utilisées pour une application topique, et notamment sous forme de solutions huileuses, alcooliques ou hydroalcooliques, de gels, d'émulsions eau-dans-huile ou huile-dans-eau ayant l'aspect d'une crème ou d'un gel, éventuellement aptes à mousser, sous forme d'aérosol, ou encore sous forme de dispersions vésiculaires contenant des lipides ioniques et/ou non ioniques. Ces formes galéniques sont préparées selon les méthodes usuelles des domaines considérés.

Les quantités des différents constituants de la composition utilisée selon l'invention sont celles classiquement utilisées dans les domaines considérés.

Cette composition peut constituer notamment une composition de nettoyage, de protection, de traitement ou de soin pour le visage, pour le cou, pour les mains ou pour le corps, (par exemple crèmes de jour, crèmes de nuit, crèmes démaquillantes, crèmes ou huiles solaires, huiles pour le corps ou le visage, laits de nettoyage, laits de démaquillage, des laits corporels), une composition de maquillage (par exemple fond de teint), une composition de bronzage artificiel, ou une composition pour le bain.

Selon une forme préférée de réalisation, la composition utilisée selon l'invention est une émulsion. Dans une telle émulsion, la proportion totale de corps gras, y compris l'huile de graine de coriandre, peut aller de 5 % à 80 % en poids, et de préférence de 5 % à 50 % en poids par rapport au poids total de la composition. Les corps gras et les émulsionnants utilisés dans la composition sous forme d'émulsion sont choisis parmi ceux classiquement utilisés dans le domaine cosmétique ou dermatologique.

Comme autres corps gras que l'huile de coriandre, utilisables dans l'invention, on peut citer les huiles minérales, les huiles végétales (huile de palme, huile de jojoba, huile d'ombellifère différente de l'huile de coriandre) et leurs dérivés hydrogénés, les huiles animales, les huiles de synthèse (polyisobutène hydrogéné, isohexadécane), les huiles siliconées (cyclométhicone) et les huiles fluorées. Comme autres corps gras, on peut encore citer les alcools gras (alcool cétylique, alcool stéarylique, alcool de lanoline), les acides gras (acide stéarique, acide palmitique) et les cires.

Les émulsionnants peuvent être présents, dans la composition, en une proportion allant de 0,3 % à 30 % en poids, et de préférence de 0,5 à 10 % en poids par rapport au poids total de la composition.

De façon connue, la composition cosmétique ou dermatologique utilisée selon l'invention peut contenir également des adjuvants habituels dans le domaine cosmétique ou dermatologique, tels que les gélifants hydrophiles ou lipophiles, les conservateurs, les antioxydants, les solvants, les parfums, les charges et les matières colorantes. Elle peut contenir aussi des actifs hydrophiles ou lipophiles et des filtres. Les quantités de ces différents adjuvants et actifs sont celles classiquement utilisées dans le domaine cosmétique ou dermatologique, et par exemple de 0,01 % à 20 % du poids total de la composition. Ces adjuvants et actifs, selon leur nature, peuvent être introduits dans la phase grasse, dans la phase aqueuse et/ou dans des vésicules lipidiques.

Comme actifs, on peut citer par exemple les actifs hydratants tels que les polyols (glycérine, propylèneglycol) et les protéines et leurs hydrolysats ; les actifs kératolytiques tels que les hydroxyacides (acide n-octanoyl-5-salicylique) ; les vitamines telles que le rétinol (vitamine A) et ses esters, l'acide ascorbique (vitamine C) et ses esters, les tocophérols (vitamine E).

Les exemples qui suivent sont donnés à titre illustratif afin de mieux faire comprendre l'invention. Les quantités indiquées sont des pourcentages en poids.

### EXEMPLE D'EXTRACTION D'HUILE DE GRAINES DE CORIANDRE

Après avoir séparé les graines de coriandre de leurs enveloppes, on les broie dans un mortier et on les presse à froid pour en extraire l'huile. On peut compléter l'extraction sur les graines broyées en procédant à une extraction à l'hexane.

L'huile obtenue est ensuite raffinée par dégommage, c'est-à-dire que l'on en élimine la gomme par décantation et filtration, puis elle est neutralisée à la soude, décolorée sur charbon actif ou par des terres décolorantes, éventuellement refroidie entre -10°C et -5°C pour séparer les fractions solides, et désodorisée.

L'huile obtenue est légèrement jaune et a les caractéristiques suivantes :
Densité relative à 20°C : 0,910 à 0,916
Indice de réfraction à 20°C : 1,466 à 1,474
Indice de saponification (mg KOH/g huile) : 186 à 198

### EXEMPLES DE COMPOSITION :

### EXEMPLE 1 : CREME DE SOIN (EMULSION HUILE-DANS-EAU)

| *Phase A :* A₁ : | |
|---|---|
| Glyceryl stearate/PEG-100 stearate (Arlacel 165 vendu par la société ICI) (émulsionnant) | 2,1 % |
| Polysorbate 60 (émulsionnant) | 0,9 % |
| Alcool cétylique | 0,5 % |
| Acide stéarique | 0,75 % |
| Acide palmitique | 0,75 % |
| Huile de palme | 2 % |
| Tricolsan (conservateur) | 0,15 % |

| A₂ : | |
|---|---|
| Huile de graines de coriandre | 14,5 % |
| Triéthanolamine | 0,4 % |
| Parfum | 0,2 % |

| *Phase B* : | |
|---|---|
| Glycérine | 3 % |
| Eau | 35,9 % |

| *Phase C* : | |
|---|---|
| Alcool de lanoline | 3 % |
| Huile minérale | 0,12 % |
| Gomme de xanthane (gélifiant) | 0,2 % |

| *Phase D* : | |
|---|---|
| Triéthanolamine (neutralisant) | 0,12 % |

| *Phase E* : | |
|---|---|
| Diazolidinyl urée (conservateur) | 0,05 % |
| Eau | qsp 100 % |

Le mode opératoire pour préparer l'émulsion consiste à chauffer les constituants de la phase A₁ à 85°C, à refroidir cette phase à 65°C et à y ajouter la phase A₂ chauffée à 65°C pour obtenir la phase A. Par ailleurs, on chauffe les constituants de la phase B à 80°C, on la refroidit à 65°C, et on ajoute la phase A dans la phase B sous agitation à 65°C. On y additionne ensuite la phase C, puis la phase D. On refroidit le mélange à 30°C sous agitation et on additionne la phase E.

### TEST D'HYDRATATION :

On a comparé le pouvoir hydratant de l'émulsion indiquée ci-dessus par rapport à la même émulsion dans laquelle l'huile de graines de coriandre a été remplacée par l'huile hybride de tournesol. Ces deux huiles se différencient par leur composition en acide gras. En effet, l'huile hybride de tournesol contient 88 % d'acide oléique qui, comme l'acide pétrosélinique, comporte une chaîne de 18 atomes de carbone, mais avec une insaturation en trans au lieu d'une insaturation en cis.

Ce test a été réalisé *in vitro* sur du *stratum corneum* isolé sur lequel a été appliquée l'émulsion à tester et dont on a déterminé la perte insensible en eau (PIE). Cette mesure permet de mettre en évidence une dégradation ou un renforcement de la barrière cutanée et indirectement l'hydratation du *stratum corneum,* car la peau devient d'autant plus séche et deshydratée qu'elle a tendance à perdre facilement son eau.

Dans un même lot de *stratum corneum,* on découpe huit échantillons de 1,3 cm² qui sont montés face externe sur le dessus de porte-échantillons. Les échantillons de *stratum corneum* sont délipidés pendant 2 heures dans un mélange de chloroforme et de méthanol (2/3 : 1/3), puis disposés pendant 12 heures dans une enceinte thermostatée afin qu'ils s'équilibrent en température (30°C) et en humidité relative (40 %). Ensuite, les échantillons de *stratum corneum* sont traités au pinceau avec la crème à tester et disposés sur un plateau tournant où chaque échantillon vient se positionner en vis-à-vis de la sonde constituant l'appareil de mesure de PIE, appelé évaporimètre et commercialisé par la société SERVOMED.

Les mesures sont effectuées aux temps To, T1 et T2, c'est-à-dire juste avant le traitement, et 1 heure et 2 heures après l'application de la crème à tester, et ces mesures sont répétées huit fois, avec, à chaque fois, un lot différent de *stratum corneum.*

Les effets observés sont quantifiés en calculant le pourcentage de variation de la moyenne des mesures de la PIE sur la zone traitée entre T1 et To ou T2 et To diminué du pourcentage de variation de la moyenne des mesures de la zone témoin constituée par du *stratum corneum* sans crème entre T1 et To ou T2 et To.

Selon cette méthode de mesure, on a déterminé le pourcentage d'évolution de la PIE des deux émulsions. Les résultats ont été les suivants :

| **Résultats de PIE** | T1 | T2 |
|---|---|---|
| Emulsion à l'huile de graines de coriandre | + 12 % | + 9 % |
| Emulsion à l'huile hybride de tournesol | + 5 % | + 5 % |

Ces résultats montrent que la PIE est meilleure avec l'émulsion contenant l'huile de graines de coriandre qu'avec l'émulsion contenant l'huile hybride de tournesol et que, par conséquent, l'émulsion de l'invention présente des propriétés hydratantes supérieures à celles de l'émulsion contenant l'huile hybride de tournesol.

### EXEMPLE 2 : LAIT CORPOREL (EMULSION HUILE-DANS-EAU)

| *Phase huileuse* : | |
|---|---|
| Glyceryl stearate/PEG-100 stearate (Arlacel 165 vendu par la société ICI) (émulsionnant) | 1 % |
| Polysorbate 60 (émulsionnant) | 0,8 % |
| Polyisobutène hydrogéné | 2 % |
| Acide stéarique | 1 % |
| Huile de graines de coriandre | 8 % |

| *Phase aqueuse* : | |
|---|---|
| Glycérine | 3 % |
| Carbomer (carbopol 941 vendu par la société Goodrich) (gélifiant) | 0,3 % |
| Triéthanolamine (neutralisant) | 0,3 % |
| Conservateur | 0,3 % |
| Eau | qsp 100 % |

On prépare l'émulsion en incorporant la phase huileuse dans la phase aqueuse sous agitation. On obtient un lait corporel qui protège la peau et l'hydrate bien.

### EXEMPLE 3 : FLUIDE DE SOIN (EMULSION HUILE-DANS-EAU)

| *Phase huileuse* : | |
|---|---|
| Méthylglucose sesquistéarate (émulsionnant) | 2 % |
| Cyclométhicone | 13 % |
| Huile de graines de coriandre | 2 % |
| Parfum | 0,2 % |
| PEG 20 méthylglucose sesquistéarate (émulsionnant) | 2 % |

| *Phase aqueuse* : | |
|---|---|
| Gomme de xanthane (gélifiant) | 0,2 % |
| Polyacrylamide/isoparaffine C13-C14/laureth-7 (Sepigel 305 vendu par la société Seppic) (gélifiant) | 0,8 % |
| Conservateur | 0,3 % |
| Eau | qsp 100 % |

On prépare l'émulsion comme dans l'exemple 2. On obtient un fluide blanc, qui assure une bonne protection à la peau.

### EXEMPLE 4 : CREME DE SOIN (EMULSION HUILE-DANS-EAU)

| *Phase huileuse* : | |
|---|---|
| Glyceryl stéarate/PEG-100 stéarate (Arlacel 165 vendu par la société ICI) (émulsionnant) | 1 % |
| PEG 20 stéarate (émulsionnant) | 1 % |
| Acide stéarique | 1 % |
| Alcool stéarylique | 2 % |
| Huile de graines de coriandre | 20 % |

| *Phase aqueuse* : | |
|---|---|
| Hydrolysat de protéines de soja | 0,2 % |
| Glycérine | 3 % |
| Carbomer (carbopol 941 vendu par la société Goodrich) (gélifiant) | 0,4 % |
| Triéthanolamine (neutralisant) | 0,4 % |
| Conservateur | 0,3 % |
| Eau | qsp 100 % |

On prépare l'émulsion comme dans l'exemple 2. On obtient une crème blanche, lisse, agréable à utiliser et apportant une bonne hydratation à la peau.

### EXEMPLE 5 : CREME DE SOIN (EMULSION EAU-DANS- HUILE)

| *Phase huileuse* : | |
|---|---|
| Polyglyceryl-4 Isostéarate/Cetyl dimethicone copolyol/Hexyl laurate (Abil WE 09 vendu par la société Goldschmidt) (émulsionnant) | 4 % |
| Isohexadécane | 5 % |
| Huile de graines de coriandre | 10 % |
| Cyclométhicone | 3,5 % |
| Acide n-octanoyl-5-salicylique | 1 % |
| Parfum | 0,15 % |

| *Phase aqueuse* : | |
|---|---|
| Glycérine | 10 % |
| Gomme de cellulose | 0,5 % |
| Sulfate de magnésium | 0,65 % |
| Conservateur | 0,3 % |
| Eau | qsp 100 % |

Pour préparer l'émulsion, on chauffe les constituants de la phase A à 80°C jusqu'à dissolution complète des différents constituants, puis on la refroidit à 65°C. On chauffe la phase B à 65°C et on la verse dans la phase A sous agitation, puis on refroidit le mélange.

On obtient une crème blanche, lisse, nourrissante, qui assure une bonne protection de la peau et évite sa deshydratation.

### EXEMPLE 6 : FOND DE TEINT (EMULSION HUILE-DANS-EAU)

| *Phase huileuse* : | |
|---|---|
| Monostéarate de glycéryle oxyéthyléné (30 OE) (Tagat S commercialisé par la société Goldschmidt) (émulsionnant) | 3 % |
| Mono-di-isostéarate de glycéryle (Tegin M commercialisé par la société Goldschmidt) (émulsionnant) | 4 % |
| Huile de graines de coriandre | 2 % |
| Huile de jojoba | 2 % |
| Cyclométhicone | 8 % |
| Oxyde de titane | 8 % |
| Oxydes de fer | 1,7 % |
| Acide stéarique | 2,4 % |
| Parfum | 0,3 % |

| *Phase aqueuse* : | |
|---|---|
| Glycérine | 3 % |
| Propylèneglycol | 3 % |
| Triéthanolamine | 1,2 % |
| Silicate de magnésium et d'aluminium hydraté | 1,5 % |
| Carboxyméthylcellulose de sodium (gélifiant) | 0,14 % |
| Conservateur | 0,3 % |
| Eau | qsp 100 % |

L'émulsion est préparée par incorporation de la phase huileuse dans la phase aqueuse sous agitation. Le fond de teint obtenu s'étale bien et a un bon pouvoir couvrant tout en apportant une bonne hydratation à la peau.

## Revendications

1. Utilisation d'une huile extraite de la graine de coriandre, comme agent hydratant dans une composition cosmétique et/ou dermatologique.

2. Utilisation selon la revendication 1, caractérisée en ce que l'huile contient au moins 40 % d'acide pétrosélinique.

3. Utilisation selon l'une quelconque des revendications précédentes, caractérisée en ce que la quantité d'huile va de 0,01 % à 50 % en poids par rapport au poids total de la composition.

4. Utilisation selon l'une quelconque des revendications précédentes, caractérisée en ce qu'elle se présente sous forme d'une émulsion.

5. Utilisation d'une huile extraite de la graine de coriandre pour préparer une pommade ou un onguent dermatologique destiné au traitement thérapeutique de la peau sèche.

6. Procédé pour hydrater la peau consistant à appliquer sur celle-ci une composition cosmétique contenant une huile extraite de la graine de coriandre.

## Claims

1. Use of an oil extracted from coriander seed, as a moisturizer in a cosmetic and/or dermatological composition.

2. Use according to Claim 1, characterized in that the oil contains at least 40% of petroselinic acid.

3. Use according to either of the preceding claims, characterized in that the amount of oil ranges from 0.01% to 50% by weight relative to the total weight of the composition.

4. Use according to any one of the preceding claims, characterized in that it is in the form of an emulsion.

5. Use of an oil extracted from coriander seed to prepare a dermatological ointment or salve intended for the therapeutic treatment of dry skin.

6. Process for moisturizing the skin, which consists in applying thereto a cosmetic composition containing an oil extracted from coriander seed.

## Patentansprüche

1. Verwendung eines aus Koriandersamen gewonnenen Öls als Hydratisierungsmittel in einer kosmetischen und/oder pharmazeutischen Zusammensetzung.

2. Verwendung nach Anspruch 1, dadurch gekennzeichnet, daß das Öl mindestens 40 % Petroselinsäure enthält.

3. Verwendung nach einem der vorhergehenden Ansprüche, dadurch gekennzeichnet, daß der Mengenanteil des Öls im Bereich von 0,01 bis 50 Gew.-%, bezogen auf das Gesamtgewicht der Zusammensetzung, liegt.

4. Verwendung nach einem der vorhergehenden Ansprüche, dadurch gekennzeichnet, daß die Zusammensetzung in Form einer Emulsion vorliegt.

5. Verwendung eines aus Koriandersamen gewonnenen Öls zur Herstellung einer dermatologischen Pomade oder Salbe, die zur therapeutischen Behandlung trockener Haut bestimmt ist.

6. Verfahren zur Hydratisierung der Haut, das darin besteht, auf die Haut eine kosmetische Zusammensetzung aufzutragen, die ein aus Koriandersamen gewonnenes öl enthält.
